# EUROPEAN PATENT SPECIFICATION

(11) **EP 3 666 256 B1**
(45) Date of publication and mention of the grant of the patent: **01.10.2025**
(21) Application number: 19215621.4
(22) Date of filing: 12.12.2019
(51) Int. Cl.: A61K 9/00, A61K 9/50

(54) **PHARMACEUTICAL COMPOSITION FOR SOLID DOSAGE FORMS**
PHARMAZEUTISCHE ZUSAMMENSETZUNG FÜR FESTE DARREICHUNGSFORMEN
COMPOSITION PHARMACEUTIQUE POUR FORMES POSOLOGIQUES SOLIDES

(30) Priority: 12.12.2018 CZ 20180700
(43) Date of publication of application: 17.06.2020
(73) Proprietor: Zentiva, K.S., 10200 Praha 10 - Dolni Mecholupy (CZ)
(72) Inventor: Zvatora, Pavel, 19800 Praha 9 (CZ); Rychecky, Ondrej, 33824 Brasy (CZ)
(74) Representative: Hartvichova, Katerina

(56) References cited:
- EP-A1- 1 637 130
- O. RYCHECKY ET AL: "(111b) Comprehensive Study of Usage of Oil Liquid Marbles in Pharma Industry | AIChE", 25 April 2018 (2018-04-25), pages 1 - 3, XP055686010, Retrieved from the Internet <URL:https://www.aiche.org/conferences/world-congress-on-particle-technology/2018/proceeding/paper/111b-comprehensive-study-usage-oil-liquid-marbles-pharma-industry> [retrieved on 20200415]
- J. EMAMI ET AL: "Formulation and optimization of solid lipid nanoparticle formulation for pulmonary delivery of budesonide using Taguchi and Box-Behnken design", 1 February 2015 (2015-02-01), pages 1 - 22, XP055685643, Retrieved from the Internet <URL:https://www.ncbi.nlm.nih.gov/pmc/articles/PMC4578209/> [retrieved on 20200415]
- BERNARD P. BINKS ET AL: "Particles at Oil-Air Surfaces: Powdered Oil, Liquid Oil Marbles, and Oil Foam", ACS APPLIED MATERIALS & INTERFACES, vol. 7, no. 26, 24 June 2015 (2015-06-24), US, pages 14328 - 14337, XP055685323, ISSN: 1944-8244, DOI: 10.1021/acsami.5b02890
- ROXANA-ELENA AVRAMESCU ET AL: "Liquid Marbles: From Industrial to Medical Applications", MOLECULES ONLINE, vol. 23, no. 5, 9 May 2018 (2018-05-09), DE, pages 1120, XP055685350, ISSN: 1433-1373, DOI: 10.3390/molecules23051120
- GUANGZHAO ZHANG ET AL: "Pickering Emulsion-Based Marbles for Cellular Capsules", MATERIALS, vol. 9, no. 7, 14 July 2016 (2016-07-14), pages 572, XP055685805, DOI: 10.3390/ma9070572

## Description

### Field of the Invention

The invention provides a pharmaceutical composition based on oil marbles, suitable for solid drug dosage forms, especially for hard capsules.

### Background Art

Liquid marbles are structures containing the interface liquid - solid - gas. These structures are formed by encapsulating small volumes of liquid in a shell formed by solid particles. The liquid core is completely separated from the surrounding environment by air pockets. In pharmaceutical science, the use of liquid marbles is currently being studied primarily in the preparation of microreactors or cell culture bioreactors.

The use of liquid marbles in the preparation of formulations of poorly water-soluble active ingredients dissolved in an oil phase was first described in the diploma thesis of Petra Jánská, 2017, "Preparation and characterization of liquid oil marbles" (ICT, FCHI). This thesis related to the preparation of particles containing an active ingredient dissolved in an oil droplet which was coated with solid particles. However, the compositions described in this work did not show satisfactory mechanical properties for use in pharmaceutical industry. For example, this work describes the force required to squeeze the liquid marbles to half of their initial diameter. At this force, irreversible deformation and destruction of the liquid marble shape occurred.

The article entitled "(111b) Comprehensive Study of Usage of Oil Liquid Marbles in Pharma Industry", AIChE by O.Rychecky et Al. published 25-04-2018, https://www.aiche.org/conferences/world-congress-on-particle-technology/2018/proceeding/paper/111b-comprehensive-study-usage-oil-liquid-marbles-pharma-industry, teaches the same as the above mentioned diploma thesis.

The article entitled "Pickering Emulsion-Based Marbles for Cellular Capsules" by GUANGZHAO ZHANG, CHAOYANG WANG, Materials, vol.9, no.7, 572, Published: 14 July 2016, DOI: 10.3390/ ma9070572, teaches oil marbles without pharmaceutically acceptable wax present.

Many poorly water-soluble active ingredients are formulated in the form of soft gelatin capsules. Commercial soft gelatin capsule formulations are based on a gelatin shell surrounding a liquid preparation. The gelatin shell is generally composed of gelatin, water, a plasticizer (glycerin or sorbitol) and optionally a colorant. This shell is sensitive to exposure to a higher relative humidity and temperature, resulting in a number of storage restrictions. For the same reason, this formulation is unsuitable for hygroscopic active ingredients.

Most commonly used liquid preparations for formulation in soft gelatin capsules are pure active ingredients (APIs) in liquid form, API solutions with a suitable carrier or solvent, such as soybean oil, fatty acid tryglycerides, polyethylene glycols and other solvents not dissolving the gelatin shell such as dimethylisosorbide, surfactants, etc.

However, these soft gelatine capsules are often difficult to swallow due to their size and weight which is necessary to achieve the required loading of the active ingredient. Optionally, the patient must swallow more than one capsule to achieve the desired therapeutic effect.

In the art, there is still a need for a simple method for preparing solid pharmaceutical dosage forms containing poorly (water-)soluble active ingredients with immediate release of the active ingredient or with controlled release of the active ingredient, while providing a sufficient patient comfort. Drug formulations allowing convenient administration make it easier for the patient to comply with the prescribed treatment regimen and increase the likelihood of compliance.

### Disclosure of the Invention

Within the framework of the present invention it has been found that the above described technical problems are solved by providing a pharmaceutical composition which is based on oil marbles, said oil marbles containing:
- a core containing an active ingredient (active pharmaceutical ingredient, API) which is dissolved, suspended or dispersed in a pharmaceutically acceptable solvent, said core further comprising a pharmaceutically acceptable wax and optionally at least one component selected from the group comprising a pharmaceutically acceptable polymer, a pharmaceutically acceptable solid polymer matrix; wherein the content of the pharmaceutically acceptable wax in the oil marbles is within the range of 1 to 65 wt.%, relative to the weight of the core, and
- a shell containing or comprised of solid particles, said solid particles comprising at least one pharmaceutically acceptable filler or binder, wherein the diameter of the oil marbles is in the range of 0.3 to 5 mm.

The oil marbles described in the present invention have at least a portion of the core in the solid state, i.e. they are "oil marbles with (partially) solid core" or "solid-core oil marbles". The core is then encapsulated by solid particles containing excipients from the group of fillers or binders acceptable for food and pharmaceutical use, optionally further containing at least one further active ingredient or other pharmaceutically acceptable excipients.

Active ingredients in solid crystalline or amorphous state (at storage temperature, e.g., laboratory temperature, and at atmospheric pressure) or in a mixture of such solid states can be formulated into the oil marbles according to the invention. It is also possible to formulate active ingredients in liquid state, as well as active ingredients usually available in the form of a suspension or dispersion. In the examples of this application, three different active ingredients are used as examples: valsartan, ibuprofen, suvorexant. These active ingredients differ significantly in structure as well as in indication, thus showing that the invention is generally applicable. It can therefore be concluded that the invention is independent of the chemical nature of the active ingredient. Preferably, the active ingredient has a molecular weight of up to 2500 g/mol, more preferably up to 1000 g/mol.

Preferably, the pharmaceutically acceptable solvent is a liquid selected from the group comprising: corn oil, sunflower oil, olive oil, coconut oil, palm oil, sesame oil, peanut oil, cottonseed oil, hydrogenated vegetable oil, mineral oil, rapeseed oil, castor oil, almond oil, soybean oil, glycerin, polyethylene glycol, polypropylene glycol, ethanol, water, and mixtures thereof. More preferably, the solvent is an oil or a mixture of oils.

Preferably, the pharmaceutically acceptable wax is a substance selected from the group comprising paraffin, carnauba wax, soy wax, ceresin, white wax, yellow wax, squalene, lanolin, cetyl palmitate, decyl oleate, glycerol monostearate, glycerol behenate, sodium lauryl sulfate, beeswax. Most preferably, the wax is beeswax or carnauba wax.

Preferably, the pharmaceutically acceptable polymer is selected from the group comprising low molecular weight polyethylene glycol (PEG), α-hydro-ω-hydroxypoly(oxyethylene)-poly(oxypropylene)-poly(oxyethylene) block copolymer (Poloxamer), acrylic acid polymer (Carbomer), bis(n-butyl)sebacate, polydextrose, betaglucan derivatives (Pullulan), 1,2,3-tri(cis-9-octadecenoyl)glycerol (Triolein), polyethylene glycol ether derivatives (Brij, Macrogol), polyoxyethylene sorbitan fatty acid ester (Polysorbate).

Preferably, the pharmaceutically acceptable solid polymer matrix is a polymer having a glass transition point temperature in the range of from 40 °C to 100 °C, more preferably selected from the group comprising Soluplus (chemically: polyvinylcaprolactam/polyvinyl acetate/polyethylene glycol copolymer), and Eudragits (chemically: copolymers of methacrylic acid esters). Of the Eudragits, Eudragit E100 and Eudragit EPO are particularly preferred (chemically: copolymers based on dimethylaminoethyl methacrylate, butyl methacrylate and methyl methacrylate in a ratio of 2:1:1).

The solid particles forming the shell of the oil marble prevent the contact of the encapsulated core with any surface or with any liquid or with core of another oil marble. Due to the shell formed by these solid particles, oil marbles do not agglomerate and the flow properties of the system increase.

The solid particles forming the shell are selected from the group comprising polymers, sugars, surfactants, sweeteners, proteins, plasticizers, inorganic excipients, organic acid salts, organic acids, antioxidants, emulsifiers, disintegrants, dyes (colorants).

Polymers include, in particular: agar, aliphatic polyesters, cellulose acetate, cellulose acetonitrate, ceratonia, ethylcellulose, cyclodextrins, guar gum, hydroxyethylcellulose, hydroxyethylmethylcellulose, hydroxypropylcellulose, hydroxypropyl starch, hypromellose (hypromellose sucralate, hypromellose phthalate), maltitol, maltodextrin, maltol, methylcellulose, pectin, polycarbophil, polydextrose, polymethacrylates, poly(methylvinyl ether/maleic anhydride), polyvinyl acetate phthalate, shellac, sodium hyaluronate, starch, xanthan gum, calcium alginate, carrageenan.

Sugars include, in particular: fructose, glucose, lactose, maltose, mannitol, sorbitol, sucrose. Surfactants include, in particular: cetylpyridinium chloride, sodium docusate, poloxamer, sodium laurylsulfate.

Sweeteners include, in particular: alitam, aspartame, dextrose, erythritol, ethylmaltol, lactitol, neohesperidine dihydrochalcone, saccharin, sodium saccharin, sodium cyclamate, sucralose, trehalose.

Proteins include, in particular: albumin, gelatin, zein.

Plasticizers include, in particular: acetyl tributyl citrate, acetyl triethyl citrate.

Inorganic excipients include, in particular: aluminum hydroxide, calcium carbonate, calcium phosphate, magnesium oxide, talc.

Salts of organic acids include, in particular: aluminum stearate, ascorbyl palmitate, magnesium stearate, potassium alginate, potassium benzoate, potassium citrate, sodium alginate, calcium stearate.

Acids include, in particular: ascorbic acid, citric acid, glycine.

Antioxidants include, in particular: butylated hydroxyanisole, butylated hydroxytoluene, citric acid, erythorbic acid, propyl gallate, sodium ascorbate.

Emulsifier may be lecithin.

Disintegrants include, in particular: calcium carboxymethylcellulose, sodium carboxymethylcellulose, cellulose (microcrystalline, powder), chitosan, croscarmellose sodium, crospovidone, povidone, sodium starch glycolate.

Dyes may be iron oxides.

The particles contained in the oil marble shell may optionally further contain at least one active ingredient (identical to the active ingredient present in the core, or different from the active ingredient present in the core).

The particles contained in the oil marble shell may optionally further contain pharmaceutically acceptable excipients.

In some embodiments, the active ingredient (contained in the core) is present in the oil marbles in the range of 0.1 to 80 wt. %, preferably in the range of 5 to 80 wt. %; solvents, waxes, solid polymer matrices and/or polymers are preferably present in a total amount of 5 to 95 wt. %.

Preferably, in some embodiments the active ingredient (contained in the core) is present in the oil marbles in the range of 0.1 to 80 wt. %, preferably in the range of 5 to 80 wt. %; solvents, waxes, solid polymer matrices and/or polymers are preferably present in a total amount of 5 to 95 wt. %, all amounts are relative to the total weight of the core. The solid particles on the surface of the oil marble preferably form up to 35 wt. % or up to 30 wt. %, more preferably up to 25 wt. % or up to 20 wt. % or up to 15 wt. % of the total weight of the oil marble.

The main advantage of oil marbles with solid core of the invention over the liquid marbles described in the prior art, including liquid oil marbles, is a higher mechanical strength and the ability to control the dissolution (release) rate of the API by the composition of the core or the shell of the oil marble with solid core. These parameters can be controlled by composition of the oil marbles with solid core, size of the oil marbles with solid core, material (solid particles) used to encapsulate the cores, or a combination of two or more of these factors.

The content of the pharmaceutically acceptable wax(es) in the oil marbles of the invention is thus preferably in an amount effective for providing the pre-determined release rate. The pre-determined release rates may include immediate release, controlled release or sustained release. Generally, the higher amount of the wax is present, the slower is the release rate.

The content of the pharmaceutically acceptable wax(es) in the oil marbles of the invention is 1 to 65 wt. %, more preferably from 1 to 30 wt. %, more preferably from 5 to 15 wt. %, relative to the weight of the core. The content of pharmaceutically acceptable polymers and/or polymer matrices is preferably in the range of 1 to 65 wt. %, more preferably from 5 to 35 wt. %, relative to the weight of the core.

Typically, the oil marbles of the present invention intended for sustained release of the active ingredient preferably contain wax(es) in an amount of more than 5 wt. %, or more than 10 wt. % and up to wt. 65% relative to the weight of the core. Furthermore, a pharmaceutically acceptable polymer and/or a pharmaceutically acceptable solid polymer matrix may be included in the oil marble, in an amount of from 1 to 45 wt. %, preferably from 3 to 25 wt. %, relative to the weight of the core.

Typically, the oil marbles of the present invention intended for immediate release of the active ingredient contain a small amount of wax(es), for example from 1 wt % up to 5 wt. % or up to 10 wt. %, relative to the weight of the core. Furthermore, a pharmaceutically acceptable polymer and/or a pharmaceutically acceptable solid polymer matrix may be included in the oil marble for immediate release, for example in an amount of 1 to 65 wt. %, more preferably in an amount of 5 to 35 wt. %, relative to the weight of the core.

The present invention is suitable for all types of API releases, including immediate release solid dosage forms (IR), controlled release solid dosage forms (modified release, MR), sustained release solid dosage forms (SR), or combinations thereof.

The ability to control the release of APIs from the oil marbles of the present invention represents a significant advantage for re-formulation of soft gelatin capsules with a liquid content into new oil-marble-based dosage forms. Using the present invention, soft gelatine capsules can be reformulated into hard gelatin capsules filled with oil marbles with solid core, which allows to reduce weight and size of the solid dosage form, thereby improving patient comfort during administration, and thus improving patient compliance.

The oil marbles with solid core of the present invention can be prepared by mixing, melt mixing, extrusion, wet granulation, fluid granulation, sieving, encapsulation. During the preparation process, the active ingredient may be melted, dissolved, suspended or dispersed in at least one pharmaceutically acceptable solvent, optionally using at least one pharmaceutically acceptable excipient, for example, to improve the solubility or dispersibility of the active ingredient. The prepared oil marbles with solid core may be filled into capsules, especially hard capsules, to form a pharmaceutical solid dosage form. Hard capsules filled with the oil marbles with solid core may be an immediate-release, modified-release, sustained-release dosage form, or combination thereof. Combination of various release regimens may be achieved, for example, by filling the capsule with several types of oil marbles with solid core having different compositions. Alternatively, the oil marbles may be tableted into a particulate dosage form - e.g., tablets or coated tablets.

During the preparation (in particular, extrusion or encapsulation step) of the oil marbles with solid core which contain a pharmaceutically acceptable wax or polymer, the temperature of the mixture for forming the core (i.e., API, solvent and wax) is close to the melting temperature of the wax or polymer, preferably differing by at most 10 °C from the melting temperature of the wax or polymer.

The final product in the form of capsules or tablets may be further packaged in a protective blister pack or in a vial. The protective packaging may, if desired, be provided with a desiccant, an oxygen absorbent, or a protective inert atmosphere (e.g., nitrogen).

A major advantage of the soft gelatin capsule drug dosage form as known in the art is the increase in adsorption and bioavailability of a number of APIs. The present invention illustrates, by way of example, that soft gelatin capsules containing ibruprofen (commercial product Ibalgin Rapidcaps 400 mg) can be reformulated into hard gelatin capsules using solid-core oil marbles, without compromising the properties of the formulation (Example 3). Using this approach, a reduction in the size of the solid dosage form was achieved, which improves patient comfort during administration. Furthermore, other negative properties of the soft gelatin capsule shell can be removed while achieving a substantially identical dissolution profile of the oil-marble-based hard capsule formulation as the original soft-capsule product. The desirable properties of soft gelatin capsules such as increased solubility and bioavailability are maintained in the oil-marble-based hard capsules, as demonstrated in the following examples.

The examples shown herein demonstrate that the process for preparation of solid-core oil marbles can be used for all types of API release, including solid immediate release (IR) dosage forms in which release of the active agent occurs immediately after administration of the formulation. According to the definition used by the European Medicines Agency, immediate release means that at least 75% of the active ingredient is released within 45 minutes under defined conditions (see Reflection paper on the dissolution specification for generic solid immediate release products with systemic action, http://www.ema.europa.eu/docs/en_GB/document_library/Scientific_guideline/2017/08/WC5 00233571.pdf).

Furthermore, by selecting a suitable solid-core oil marble composition within the scope of the present invention, controlled release solid dosage forms (MR) and sustained release solid dosage forms (SR), or combinations thereof, can be prepared.

In dosage forms containing solid-core oil marbles, the homogenization of the active ingredient in the solution or melt phase ensures uniformity of content of the active ingredient in the final product, so that the dosage forms can be produced with very high (up to 80%) drug loading, as well as with very low (even below 5% by weight) drug loading.

### Brief Description of Drawings

Figure 1: Release of valsartan from solid-core oil marbles, as depending on the beeswax content of the composition: a) 4.9% wax; b) 10.2% wax; c) 19.8% wax; d) 29.6% wax.
Figure 2: Release of valsartan from a solid-core oil marble formulation - an example of an immediate release formulation.
Figure 3: a) Release of ibuprofen from a solid-core oil marble formulation; b) release of ibuprofen from a commercial preparation Ibuprofen Rapidcaps^{®} (soft gelatin capsules, comparative example).
Figure 4: Release of suvorexant from solid-core oil marble formulation: a) with a 5.5 wt. % beeswax content; b) with 0% beeswax.
Figure 5: Strength of solid-core oil marbles depending on wax content and type: a) Carnauba wax; b) beeswax; c) soybean wax.

### Examples of carrying out the Invention

The invention is illustrated by the following examples. These examples, which show the preparation of solid dosage forms of ibuprofen, valsartan and suvorexant, are merely illustrative and should not be construed as limiting the scope of the invention.

### Experimental methods:

### Preparation of solid-core oil marbles - general procedure

The active ingredient was dissolved in oil with stirring, followed by the addition of wax or polymer to the desired concentration, and the whole mixture was heated to melt and homogenize. The melt was then extruded dropwise onto a pre-prepared powder in a dish using a syringe. The melt temperature was maintained just above the melting point, so that upon formation of the droplet and its contact with the solid particles, the mixture immediately solidified and therefore no excessive absorption of the solid particles into the liquid core occurred. After the solidification of the droplets, the whole dish is stirred in order to coat the whole surface of the resulting marbles by the solid particles.

### Measurement of dissolved amount of active ingredients in oils

The highest dissolved amount of the active ingredient in oil was determined using a Crystal 16 instrument, which records the turbidity of the solution. Accurate amounts of drug and oil were weighed into 1 ml vials, and the mixture was heated slowly (3 °C/min) in the apparatus and stirred constantly at 700 rpm. The solution was heated withing the range of 20 to 100 °C. After reaching 100 °C, the solution was left for 30 min at this temperature, and cooling occurred at the same rate of temperature change.

### Dissolution of ibuprofen potassium from solid-core oil marbles

The dissolution kinetics was continuously measured on a µDISS Profiler^{™} - Pion UV/Vis spectrophotometer with bottom stirring using electromagnetic stirrers. Dissolution tests were performed in 900 ml of phosphate buffer solution at pH = 7.2, with constant stirring at 200 rpm for a specified time. Samples were measured at spacing intervals, 10 samples in one batch.

### Dissolution of valsartan from solid-core oil marbles

The dissolution kinetics was continuously measured on a µDISS Profiler^{™} - Pion UV/Vis spectrophotometer with bottom stirring using electromagnetic stirrers. Dissolution tests were performed in 200 ml phosphate buffer solution at pH = 7.2, with constant stirring at 200 rpm for a specified time. Samples were measured at spacing intervals, 10 samples in one batch.

### Dissolution of suvorexant from solid-core oil marbles

The dissolution kinetics was continuously measured on a µDISS Profiler^{™} - Pion UV/Vis spectrophotometer with bottom stirring using electromagnetic stirrers. Dissolution tests were performed in 200 ml buffer solution pH = 1.5, with constant stirring at 200 rpm for a specified time. Samples were measured at spacing intervals, 10 samples in one batch.

### Measurement of mechanical properties of solid-core oil marbles

Mechanical stability tests were measured on a Brookfield TexturePro CT3 with a cylindrical probe (diameter = 4 mm), according to the manufacturer's instructions. The probe speed was 0.1 mm/s. A solid-core oil marble was positioned on a stationary plate and deformed.

### Example 1

Preparation of solid-core oil marbles and measurement of the effect of the amount of wax on the dissolution rate of valsartan

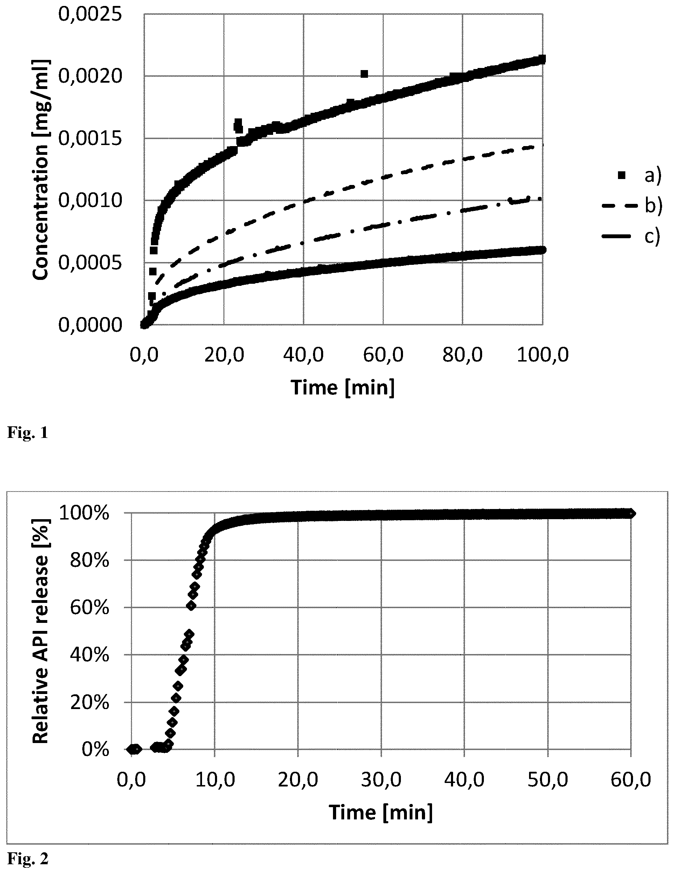

The weighed amount of the mixture of valsartan, rapeseed oil and wax (according to the compositions shown in Table 1) was heated to 72 °C on an electromagnetic stirrer and stirred at 150 rpm. The exact composition of the marbles is shown in Table 1. During stirring, a Petri dish with mannitol was prepared to which the melt was added dropwise, using a 1 ml syringe with a 0.8 mm needle. Subsequently, 200 ml of phosphate buffer pH 7.2 was provided in a beaker, and an exact amount of solid-core oil marbles containing valsartan was weighed into the beaker. The UV/Vis spectrophotometer µDISS Profiler^{™}-Pion measured the absorbance at 250 nm every 10 seconds. Figure 1 shows that the concentration of beeswax in solid-core oil marbles influences the release rate of the active ingredient, namely a higher concentration of beeswax results in a slower release.

The course of valsartan release from the prepared marbles is shown in Figure 1.

**Table 1: Composition of individual solid-core oil marbles for measurement of release rate**

| sample | Valsartan (%) | rapeseed oil (%) | beeswax (%) |
|---|---|---|---|
| d) | 9.0 | 61.4 | 29.6 |
| c) | 9.1 | 71.0 | 19.8 |
| b) | 9.0 | 80.8 | 10.2 |
| a) | 9.1 | 85.9 | 4.9 |

### Example 2 (not according to the claims)

Preparation of solid-core oil marbles containing valsartan with immediate release The melt composition was as follows (weight %): valsartan 10%, poloxamer 188 20%, castor oil 70%, and the mixture was heated to 60 °C. Subsequently, marbles were prepared by dropwise dripping (needle diameter 0.8 mm) into a powdered lactose bed. Dissolution was measured in 200 ml phosphate buffer pH 7.2 on a µDISS Profiler^{™}-Pion UV/Vis spectrophotometer. The valsartan release pattern is shown in Figure 2, and corresponds to an immediate release formulation.

### Example 3

### Preparation of solid-core oil marbles containing ibuprofen

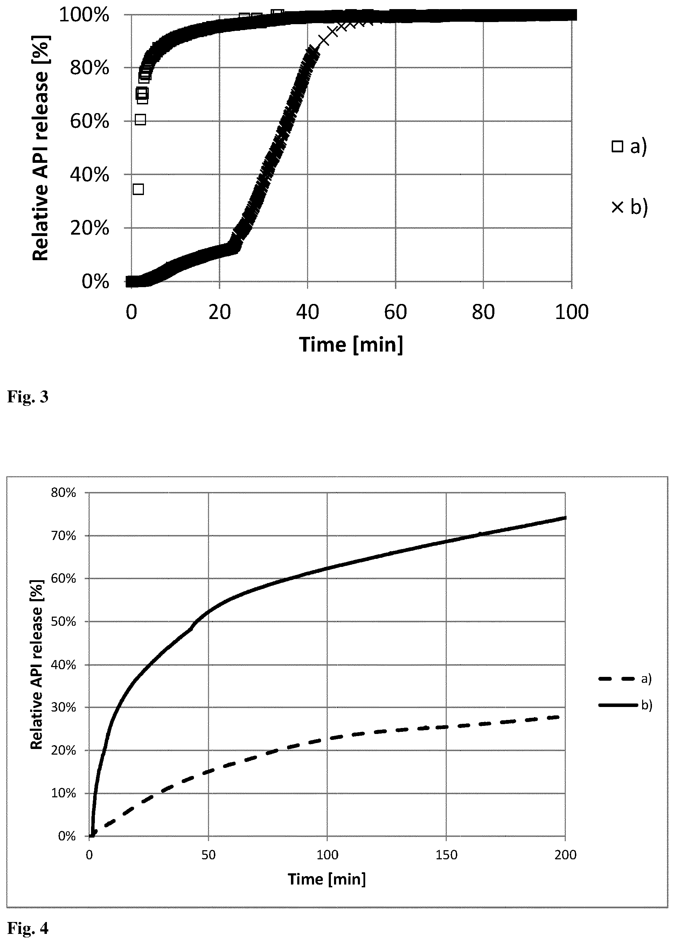

Ibuprofen potassium salt was prepared from a mixture of ethanol with dissolved potassium hydroxide and of ibuprofen in a stoichiometric ratio. After mixing, the ethanol was evaporated overnight in a vacuum evaporator at 50 °C. A mixture of rapeseed oil, beeswax, ibuprofen and ibuprofen potassium was then prepared and added dropwise onto a powdered mannitol bed. The prepared solid-core oil marbles were filled into hard gelatin capsules of size 00 (23.5 x 8.5 mm). Dissolution rate measurements were performed on a µDISS Profiler ^{™}-Pion UV/Vis spectrophotometer in 900 ml of phosphate buffer pH 7.2. The commercial drug Ibuprofen Rapidcaps (15x10 mm oval soft gelatin capsules) was measured under the same conditions. A comparison of the release rate from both formulations is shown in Figure 3. The composition according to the invention had a faster release rate than the commercially available product.

| composition | wt. % |
|---|---|
| Ibuprofen | 43.4 |
| Ibuprofen potassium salt | 26.5 |
| beeswax | 10.0 |
| rapeseed oil | 20.1 |

### Example 4

### Preparation of solid-core oil marbles with the active ingredient (sample 2 not according to the claims)

A weighted amount of suvorexant was dissolved in rapeseed oil in a beaker, then the other ingredients were added (see table). The mixture containing beeswax was heated to 67 °C, the wax-free mixture was heated to 62 °C. Subsequently, the mixtures were added dropwise into lactose powder bed, using a 22G steel or PTFE needle. Suvorexant release rate measurements were performed on a µDISS Profiler^{™}-Pion UV/Vis spectrophotometer in 200 ml of acid buffer pH 1.5 at 280 nm. The release rate is influenced by the concentration of beeswax in the marble composition; the composition only with poloxamer is suitable for rapid release. The release rates are shown in Figure 4.

| sample | Suvorexant (%) | rapeseed oil (%) | poloxamer (%) | beeswax (%) |
|---|---|---|---|---|
| 1 | 4.8 | 59.4 | 30.3 | 5.5 |
| 2 | 7.0 | 60.7 | 32.3 | - |

### Example 5

### Preparation of solid-core oil marbles for mechanical properties tests

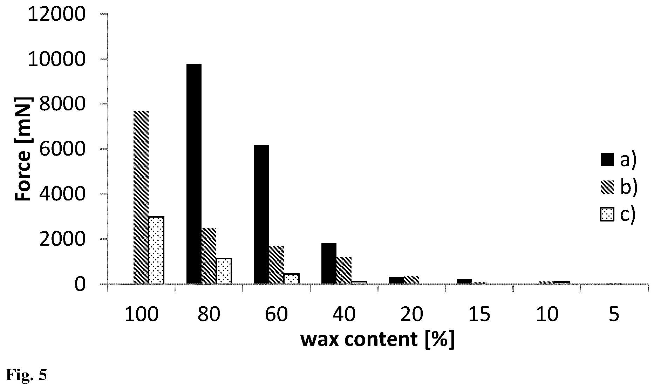

The mechanical properties of solid-phase oil marbles were measured for various mixtures of rapeseed oil and beeswax or soybean wax or carnauba wax, these waxes having different melting points. The mixtures were at a concentration of 5%, 10%, 15%, 20%, 40%, 60%, 80% and 100% (wt. %) of wax in the solid core of oil marbles, the remainder being rapeseed oil. The mixture was heated to the melting point of the individual waxes and the mixture was homogenized by stirring at 200 rpm with an electromagnetic stirrer. Solid-core oil marbles were coated in mannitol. The mechanical stability measurement was then carried out with the Brookfield TexturePro CT3. We observed the upper yield point of the marbles and the results for the individual compositions are shown in Figure 5. A higher proportion of beeswax in the composition indicates a higher resistance to mechanical stress. The maximum limit of the measuring equipment was exceeded for marbles with 100% content of carnauba wax. It can be seen that a higher wax concentration leads to a higher yield strength value. However, at the same wax concentration, it is apparent that carnauba wax has the highest yield strength values compared to soybean or beeswax. As described above, mechanical properties suitable for filling hard gelatin capsules with solid-core oil marbles can be achieved, as is the case with conventional dosage forms such as pellets or mini-tablets.

Experiments have shown that the amount of wax in the matrix can control the release from the formulation (Figure 1). Using a suitable combination of wax and oil, solid-core oil marble formulations can be prepared with immediate release of the drug (Figure 2 and Figure 3), or controlled or sustained release. Water-soluble substances can also be formulated as described (Figure 4). In the present embodiments, an active ingredient was present in the range of content of 5 to 80 wt. %. All prepared marbles had a size ranging from 0.3 to 3 mm. The wax content and type of wax significantly affects the resulting mechanical properties of the solid-core oil marbles (Figure 5).

### Example 6: Mechanical properties of solid-core marbles (samples with no wax in the core are not according to the claims)

The mechanical properties, expressed as Young's modulus, were measured for various types of solid-core oil marbles. Young's modulus of elasticity was obtained as the slope of the linear part of the dependency of load (mN) on displacement (mm) during initial compression.

The mechanical stability measurement (dependency of load (mN) on displacement (mm)) was carried out with the Brookfield TexturePro CT3.

First, Young's modulus was determined for solid-core oil marbles containing rapeseed oil and various prooportions of wax in the core, and lactose in the shell. The obtained values of the slope corresponding to Young's modulus are shown in the following table:

| | Beeswax | Carnauba wax | Soya wax |
|---|---|---|---|
| Amount of wax in the core | | | |
| 20 % | 729.27 | 992.85 | 926.09 |
| 15 % | 420.63 | 737.10 | 765.589 |
| 10 % | 226.44 | 259.39 | 366.928 |
| 5% | 130.99 | 118.47 | 308.69 |
| 0 % (no wax present) | 83.601 | | |

The measurement was carried out with solid-core oil marbles containing an active ingredient, according to Examples 3 and 4, with the following results:

| Sample | slope corresponding to Young's modulus |
|---|---|
| Example 3 | 731.9 |
| Example 4, Sample 1 | 242.4 |
| Example 4, Sample 2 | 1491.5 |

## Claims

1. Oil marbles for pharmaceutical compositions, comprising:
- a core containing an active ingredient which is dissolved, suspended or dispersed in a pharmaceutically acceptable solvent, said core further comprising a pharmaceutically acceptable wax and optionally at least one component selected from the group comprising a pharmaceutically acceptable polymer, a pharmaceutically acceptable solid polymer matrix; wherein the content of the pharmaceutically acceptable wax in the oil marbles is within the range of 1 to 65 wt. %, relative to the weight of the core, and
- a shell containing or comprised of solid particles, said solid particles comprising at least one pharmaceutically acceptable filler or binder,
wherein the diameter of the oil marbles is in the range of 0.3 to 5 mm.

2. Oil marbles according to claim 1, wherein the pharmaceutically acceptable solvent is selected from the group comprising: corn oil, sunflower oil, olive oil, coconut oil, palm oil, sesame oil, peanut oil, cottonseed oil, hydrogenated vegetable oil, mineral oil, rapeseed oil, castor oil, almond oil, soybean oil, glycerin, polyethylene glycol, polypropylene glycol, ethanol, water, and mixtures thereof.

3. Oil marbles according to any one of the preceding claims, wherein the pharmaceutically acceptable wax is selected from the group comprising: paraffin, carnauba wax, soybean wax, ceresin, white wax, yellow wax, squalene, lanolin, cetyl palmitate, decyl oleate, glycerol monostearate, glycerol behenate, sodium lauryl sulfate, beeswax.

4. Oil marbles according to any one of the preceding claims, wherein the pharmaceutically acceptable polymer is selected from the group comprising: low molecular weight polyethylene glycol, α-hydro-ω-hydroxypoly(oxyethylene)-poly(oxypropylene)-poly(oxyethylene) block copolymer, acrylic acid polymer, bis(n-butyl)sebacate, polydextrose, betaglucan derivatives, 1,2,3-tri(cis-9-octadecenoyl)glycerol, polyethylene glycol ether derivatives, polyoxyethylene sorbitan fatty acid ester.

5. Oil marbles according to any one of the preceding claims, wherein the pharmaceutically acceptable solid polymer matrix is a polymer having a glass transition point temperature in the range of from 40 °C to 100 °C, preferably selected from the group comprising polyvinylcaprolactam/polyvinyl acetate/polyethylene glycol copolymer and copolymers of methacrylic acid esters.

6. Oil marbles according to any one of the preceding claims, wherein the active ingredient is contained in the oil marbles within the range of 0.1 to 80 wt. %, preferably in the range of 5 to 80 wt. %; solvents, waxes, solid polymer matrices and/or polymers are preferably present in a total amount of 5 to 95 wt. %, , relative to the weight of the core.

7. Oil marbles according to any one of claims 1 to 6, wherein the diameter of the oil marbles is in the range of 0.3 to 3 mm, preferably 0.3 to 1 mm.

8. Oil marbles according to any one of claims 1 to 7, wherein the content of the pharmaceutically acceptable wax in the oil marbles is within the range of 1 to 30 wt. %, preferably 5 to 15 wt. %, relative to the weight of the core.

9. Oil marbles according to any one of claims 1 to 7, wherein the content of pharmaceutically acceptable polymers and/or solid polymer matrices is within the range of 1 to 65 wt. %, preferably 5 to 35 wt. %, relative to the weight of the core.

10. Oil marbles according to any one of claims 1 to 7, wherein the pharmaceutically acceptable wax in the oil marbles is contained in an amount effective for a pre-determined release rate of the active ingredient.

11. Oil marbles according to any one of claims 1 to 7, wherein the content of wax is more than 5 wt. %, preferably more than 10 wt. %, relative to the weight of the core, and the oil marbles optionally contain also the pharmaceutically acceptable polymer and/or solid polymer matrix in an amount of 1 to 45 wt. %, relative to the weight of the core.

12. Oil marbles according to any one of claims 1 to 7, wherein the oil marbles contain up to 10 wt. % of wax or up to 5 wt. % of wax, relative to the weight of the core, and contain the pharmaceutically acceptable polymer and/or solid polymer matrix in an amount of 1 to 65 wt. %, relative to the weight of the core.

13. A pharmaceutical composition, which contains the oil marbles according to any one of the claims 1 to 12 in a hard capsule, preferably in a hard gelatin capsule.

14. A pharmaceutical composition, which contains the oil marbles according to any one of the claims 1 to 12, wherein the pharmaceutical composition is in the form of a particular pharmaceutical dosage form such as a tablet or a coated tablet.

## Patentansprüche

1. Ölmurmeln für pharmazeutische Zusammensetzungen, enthaltend:
- einen Kern, der einen in einem pharmazeutisch verträglichen Lösungsmittel gelösten, suspendierten oder dispergierten Wirkstoff enthält, wobei der Kern zusätzlich ein pharmazeutisch verträgliches Wachs und optional mindestens eine Komponente aus der Gruppe enthaltend ein pharmazeutisch verträglichen Polymer und eine pharmazeutisch verträgliche feste Polymermatrix enthält; wobei der Gehalt des pharmazeutisch verträglichen Wachses in den Ölmurmeln im Bereich von 1 bis 65 Gew.-%, bezogen auf das Gewicht des Kerns, liegt, und
- eine Hülle, die feste Partikel enthält oder daraus besteht, wobei die festen Partikel mindestens einen pharmazeutisch verträglichen Füllstoff oder ein Bindemittel enthalten,
wobei der Durchmesser der Ölmurmeln im Bereich von 0,3 bis 5 mm liegt.

2. Ölmurmeln nach Anspruch 1, wobei das pharmazeutisch verträgliche Lösungsmittel ausgewählt ist aus der Gruppe enthaltend: Maisöl, Sonnenblumenöl, Olivenöl, Kokosöl, Palmöl, Sesamöl, Erdnussöl, Baumwollsamenöl, gehärtetem Pflanzenöl, Mineralöl, Rapsöl, Rizinusöl, Mandelöl, Sojaöl, Glycerin, Polyethylenglykol, Polypropylenglykol, Ethanol, Wasser und Mischungen davon.

3. Ölmurmeln nach einem der vorhergehenden Ansprüche, wobei das pharmazeutisch verträgliche Wachs ausgewählt ist aus der Gruppe enthaltend: Paraffin, Carnaubawachs, Sojawachs, Ceresin, Weißwachs, Gelbwachs, Squalen, Lanolin, Cetylpalmitat, Decyloleat, Glycerinmonostearat, Glycerinbehenat, Natriumlaurylsulfat, Bienenwachs.

4. Ölmurmeln nach einem der vorhergehenden Ansprüche, wobei das pharmazeutisch verträgliche Polymer ausgewählt ist aus der Gruppe bestehend aus: niedermolekularem Polyethylenglykol, α-Hydro-ω-hydroxypoly(oxyethylen)-poly(oxypropylen)-poly(oxyethylen)-blockcopolymer, Acrylsäurepolymer, Bis(n-butyl)sebacat, Polydextrose, Betaglucanderivaten, 1,2,3-Tri(cis-9-octadecenoyl)glycerin, Polyethylenglykoletherderivaten, Polyoxyethylensorbitanfettsäureester.

5. Ölmurmeln nach einem der vorhergehenden Ansprüche, wobei die pharmazeutisch verträgliche feste Polymermatrix ein Polymer mit einer Glasübergangstemperatur im Bereich von 40 °C bis 100 °C ist, vorzugsweise ausgewählt aus der Gruppe bestehend aus Polyvinylcaprolactam/Polyvinylacetat/Polyethylenglykol-Copolymer und Copolymeren von Methacrylsäureestern.

6. Ölmurmeln nach einem der vorhergehenden Ansprüche, wobei der Wirkstoff in den Ölmurmeln im Bereich von 0,1 bis 80 Gew.-%, vorzugsweise im Bereich von 5 bis 80 Gew.-%, enthalten ist; Lösungsmittel, Wachse, feste Polymermatrices und/oder Polymere sind vorzugsweise in einer Gesamtmenge von 5 bis 95 Gew.-%, bezogen auf das Gewicht des Kerns, enthalten.

7. Ölmurmeln nach einem der Ansprüche 1 bis 6, wobei der Durchmesser der Ölmurmeln im Bereich von 0,3 bis 3 mm, vorzugsweise 0,3 bis 1 mm, liegt.

8. Ölmurmeln nach einem der Ansprüche 1 bis 7, wobei der Gehalt an pharmazeutisch verträglichem Wachs in den Ölmurmeln im Bereich von 1 bis 30 Gew.-%, vorzugsweise 5 bis 15 Gew.-%, bezogen auf das Gewicht des Kerns, liegt.

9. Ölmurmeln nach einem der Ansprüche 1 bis 7, wobei der Gehalt an pharmazeutisch verträglichen Polymeren und/oder festen Polymermatrizes im Bereich von 1 bis 65 Gew.-%, vorzugsweise 5 bis 35 Gew.-%, bezogen auf das Gewicht des Kerns, liegt.

10. Ölmurmeln nach einem der Ansprüche 1 bis 7, wobei das pharmazeutisch verträgliche Wachs in den Ölmurmeln in einer Menge enthalten ist, die eine vorbestimmte Freisetzungsrate des Wirkstoffs gewährleistet.

11. Ölmurmeln nach einem der Ansprüche 1 bis 7, wobei der Wachsgehalt mehr als 5 Gew.-%, vorzugsweise mehr als 10 Gew.-%, bezogen auf das Gewicht des Kerns, beträgt, und die Ölmurmeln enthalten optional auch das pharmazeutisch verträgliche Polymer und/oder die feste Polymermatrix in einer Menge von 1 bis 45 Gew.-%, bezogen auf das Gewicht des Kerns.

12. Ölmurmeln nach einem der Ansprüche 1 bis 7, wobei die Ölmurmeln bis zu 10 Gew.-% Wachs oder bis zu 5 Gew.-% Wachs, bezogen auf das Gewicht des Kerns, enthalten, und das pharmazeutisch verträgliche Polymer und/oder die feste Polymermatrix in einer Menge von 1 bis 65 Gew.-%, bezogen auf das Gewicht des Kerns, enthalten.

13. Eine pharmazeutische Zusammensetzung, die die Ölmurmeln nach einem der Ansprüche 1 bis 12 in einer Hartkapsel, vorzugsweise einer Hartgelatinekapsel, enthält.

14. Eine pharmazeutische Zusammensetzung, die die Ölmurmeln nach einem der Ansprüche 1 bis 12 enthält, wobei die pharmazeutische Zusammensetzung in Form einer bestimmten pharmazeutischen Darreichungsform, wie z. B. einer Tablette oder eines Dragees, vorliegt.

## Revendications

1. Billes d'huile pour compositions pharmaceutiques, comprenant :
- un noyau contenant un principe actif dissous, en suspension ou dispersé dans un solvant pharmaceutiquement acceptable, ledit noyau comprenant également une cire pharmaceutiquement acceptable et éventuellement au moins un composant choisi dans le groupe comprenant un polymère pharmaceutiquement acceptable, une matrice polymère solide pharmaceutiquement acceptable ; la teneur en cire pharmaceutiquement acceptable des billes d'huile étant comprise entre 1 et 65 % en poids par rapport au poids du noyau ; et
- une enveloppe contenant ou constituée de particules solides, lesdites particules solides comprenant au moins une charge ou un liant pharmaceutiquement acceptable,
le diamètre des billes d'huile étant compris entre 0,3 et 5 mm.

2. Billes d'huile selon la revendication 1, où le solvant pharmaceutiquement acceptable est choisi dans le groupe comprenant : l'huile de maïs, l'huile de tournesol, l'huile d'olive, l'huile de coco, l'huile de palme, l'huile de sésame, l'huile d'arachide, l'huile de coton, l'huile végétale hydrogénée, l'huile minérale, l'huile de colza, l'huile de ricin, l'huile d'amande, l'huile de soja, la glycérine, le polyéthylène glycol, le polypropylène glycol, l'éthanol, l'eau et leurs mélanges.

3. Billes d'huile selon l'une quelconque des revendications précédentes, où la cire pharmaceutiquement acceptable est choisie dans le groupe comprenant : la paraffine, la cire de carnauba, la cire de soja, la cérésine, la cire blanche, la cire jaune, le squalène, la lanoline, le palmitate de cétyle, l'oléate de décyle, le monostéarate de glycérol, le béhénate de glycérol, le laurylsulfate de sodium et la cire d'abeille.

4. Billes d'huile selon l'une quelconque des revendications précédentes, où le polymère pharmaceutiquement acceptable est choisi dans le groupe comprenant : le polyéthylène glycol de faible poids moléculaire, le copolymère séquencé α-hydro-ω-hydroxypoly(oxyéthylène)-poly(oxypropylène)-poly(oxyéthylène), le polymère d'acide acrylique, le sébaçate de bis(n-butyle), le polydextrose, les dérivés de bêta-glucane, le 1,2,3-tri(cis-9-octadécénoyl)glycérol, les dérivés de polyéthylène glycol éther, l'ester d'acide gras de polyoxyéthylène sorbitan.

5. Billes d'huile selon l'une quelconque des revendications précédentes, où la matrice polymère solide pharmaceutiquement acceptable est un polymère dont la température de transition vitreuse est comprise entre 40 °C et 100 °C, de préférence choisi dans le groupe comprenant le copolymère polyvinylcaprolactame/acétate de polyvinyle/polyéthylène glycol et les copolymères d'esters d'acide méthacrylique.

6. Billes d'huile selon l'une quelconque des revendications précédentes, où l'ingrédient actif est contenu dans les billes d'huile dans la plage de 0,1 à 80 % en poids, de préférence dans la plage de 5 à 80 % en poids ; les solvants, les cires, les matrices polymères solides et/ou les polymères sont de préférence présents dans une quantité totale de 5 à 95 % en poids, par rapport au poids du noyau.

7. Billes d'huile selon l'une quelconque des revendications 1 à 6, dont le diamètre est compris entre 0,3 et 3 mm, de préférence entre 0,3 et 1 mm.

8. Billes d'huile selon l'une quelconque des revendications 1 à 7, dont la teneur en cire pharmaceutiquement acceptable est comprise entre 1 et 30 % en poids, de préférence entre 5 et 15 % en poids, par rapport au poids du noyau.

9. Billes d'huile selon l'une quelconque des revendications 1 à 7, dont la teneur en polymères et/ou matrices polymères solides pharmaceutiquement acceptables est comprise entre 1 et 65 % en poids, de préférence entre 5 et 35 % en poids, par rapport au poids du noyau.

10. Billes d'huile selon l'une quelconque des revendications 1 à 7, dont la cire pharmaceutiquement acceptable est présente en quantité efficace pour une vitesse de libération prédéterminée du principe actif.

11. Billes d'huile selon l'une quelconque des revendications 1 à 7, où la teneur en cire est supérieure à 5 % en poids, de préférence supérieure à 10 % en poids, par rapport au poids du noyau, et les billes d'huile contiennent éventuellement également le polymère pharmaceutiquement acceptable et/ou la matrice polymère solide en une quantité de 1 à 45 % en poids, par rapport au poids du noyau.

12. Billes d'huile selon l'une quelconque des revendications 1 à 7, où les billes d'huile contiennent jusqu'à 10 % en poids de cire ou jusqu'à 5 % en poids de cire, par rapport au poids du noyau, et contiennent le polymère pharmaceutiquement acceptable et/ou la matrice polymère solide en une quantité de 1 à 65 % en poids, par rapport au poids du noyau.

13. Composition pharmaceutique contenant les billes d'huile selon l'une quelconque des revendications 1 à 12 dans une capsule dure, de préférence une capsule de gélatine dure.

14. Composition pharmaceutique, qui contient les billes d'huile selon l'une quelconque des revendications 1 à 12, où la composition pharmaceutique est sous la forme d'une forme posologique pharmaceutique particulaire telle qu'un comprimé ou un comprimé enrobé.
